# EUROPEAN PATENT SPECIFICATION

(11) **EP 3 679 819 B1**
(45) Date of publication and mention of the grant of the patent: **31.01.2024**
(21) Application number: 18835303.1
(22) Date of filing: 28.06.2018
(51) Int. Cl.: A41D 13/08, A61F 5/058

(54) **WRIST PROTECTING BAND**
HANDGELENKSCHUTZBAND
BANDE DE PROTECTION DE POIGNET

(30) Priority: 21.07.2017 KR 20170092915
(43) Date of publication of application: 15.07.2020
(73) Proprietor: Lee, Sang Jin, Jinju-si, Gyeongsangnam-do 52717 (KR)
(72) Inventor: Lee, Sang Jin, Jinju-si, Gyeongsangnam-do 52717 (KR)
(74) Representative: Ter Meer Steinmeister & Partner
(86) International application number: PCT/KR2018/007339
(87) International publication number: WO 2019/017609

(56) References cited:
- EP-A1- 1 369 080
- KR-A- 20000 074 622
- KR-A- 20120 127 814
- KR-A- 20160 147 231
- KR-A- 20160 147 231
- US-A- 5 921 949
- US-A1- 2003 135 280
- US-A1- 2008 039 765
- US-A1- 2013 012 856

## Description

### TECHNICAL FIELD

The present disclosure relates to a wrist protecting band. More particularly, the present disclosure relates to a wrist protecting band, in which compression pads are used to compress only the ulna area and the radius area of the wrist toward the center of the wrist, thereby enabling the wrist protecting band to prevent side effects, such as hand tingling or hand swelling, and reliably perform a wrist protecting function. Coupling force between compression members pivotably coupled to each other is adjusted by a user's manipulation so as to adjust a compression load applied to the ulna area and radius area of the wrist, thereby enabling the wrist protecting band to be more conveniently and optimally used according to user requirements. The overall diameter of the wrist protecting band may be increased or reduced, so that the process of attaching or detaching the wrist protecting band to or from the wrist may be performed more conveniently. It is possible to compress or decompress the wrist by pivoting the compression members in a simple manner using a dial-type manipulator while adjusting the strength of compression in a simple manner using the manipulator.

### BACKGROUND ART

Regarding the joints of an arm of a human body, the radius and the ulna constitute the forearm, eight (8) carpal bones constitute the wrist, five (5) metacarpal joints by which pivoting, flexing, and extension are enabled constitute the palm, and phalangeal bones constitute each finger while providing a function of gripping an object to the finger.

Such joints may be injured by repeated and continuous excessive use thereof due to operations of holding objects with hands or due to activities. In some cases, injured joints may not be completely healed and continuous pain may occur.

Therefore, wrist protecting bands respectively attached to a wrist of a human body to protect such joints and bones have been proposed and used.

In general, a wrist protecting band is configured to be attached to a frail joint portion of a person like an elbow protector, an ankle protector, a forearm protector, and the like.

A typical wrist protector of the related art has the shape of a band surrounding a wrist, and is placed along the outer surface of the wrist to uniformly compress the wrist and portions adjacent to the wrist. The wrist protector serves to protect the wrist by compressing the wrist and prevent the wrist from being excessively bent.

However, because a wrist protector of the related art uniformly compresses the entire area of the wrist, if the wrist protector has been attached to the wrist for an extended period of time, blood may not flow through the wrist artery properly, thereby causing the hand to tingle or swell, which is problematic. Due to the problem, such as hand tingling, the use of the wrist protector is limited. For example, a user may remove the wrist protector during or before driving. Accordingly, the wrist may be exposed to the danger of an injury during a variety of exercises or activities, instead of being safely protected.
KR 2016-0147231 A describes a detachable tightening control pad structure comprising: a base pad detachably attachable to an arbitrary object; a winding reel attached to a base pad capable of switching between a winding mode in which a loop-shaped fastening wire is able to be wound from an outside to an inside by an one-side revolution, and a pull-out mode in which a fastening wire wound inside is released and is able to manually be pulled in which the fastening wire wound inside is released and is able to manually be pulled out; and a wire guide section attached to an arbitrary object, guiding a movement of the fastening wire whose length is adjusted through winding and pulling-out of the winding reel.
EP 1 369 080 A describes a cuff of a wrist-mount blood pressure monitor including an inflatable bag to which a predetermined quantity of fluid is supplied in order to press predetermined artery of a wrist, and a fixing member for mounting the inflatable bag on the wrist. The fixing member has a first half split ring-shaped fixing tool and a second half split ring-shaped fixing tool having an approximately ring shape with rigidity for preventing elastic deformation in a state where the inflatable bag is inflated.
US 2008/039765 A1 describes an apparatus for disposition on appendage, such as an arm or leg, is capable of encircling and applying opposing forces selectively thereto. A C-shaped component receives one side of the appendage, a second C-shaped component receives the other side of the appendage, and a tensioning system draws the two components together for selective application of pressure to the appendage. US5921949 discloses a wrist proetcting band according to the preamble of claim 1.

### DISCLOSURE Technical Problem

The present disclosure has been made in consideration of the above-described problems occurring in the related art, and an object of the present disclosure is to provide a wrist protecting band able to compress only an ulna area and a radius area of the wrist toward the center of the wrist using compression pads in order to prevent side effects, such as hand tingling or hand swelling. The use of the compression pads can also supplement fixing force of the wrist in order to reliably perform a wrist protecting function of, e.g. preventing the wrist from being excessively bent and reducing pain in the wrist.

Another object of the present disclosure is to provide a wrist protecting band in which the compression members are configured to be pivotably coupled to each other and the coupling force between the coupling members is adjusted by a user's manipulation, so that a compression load applied to the ulna area and radius area of the wrist can be adjusted. Accordingly, the wrist protecting band can be more conveniently and optimally used according to user requirements.

Another object of the present disclosure is to provide a wrist protecting band in which the overall diameter of the wrist protecting band may be increased or reduced using the compression members coupled to each other to be pivotable and a connecting member, so that the process of attaching or detaching the wrist protecting band to or from the wrist can be performed more conveniently. In addition, it is possible to adjust the size of the internal diameter by changing the size of the connecting member without having to change the compression members, thereby facilitating the management of components and the fabrication of the wrist protecting band.

Another object of the present disclosure is to provide a wrist protecting band enabling a user to compress or decompress the wrist by pivoting the compression members in a simple manner using a dial-type manipulator while adjusting the strength of compression in a simple manner using the manipulator.

Another object of the present disclosure is to provide a wrist protecting band enabling the user to adjust the positions and thicknesses of the compression pads compressing the ulna area and the radius area of the wrist according to user requirements, so that the user can more conveniently use the wrist protecting band by modifying the wrist protecting band in an optimal state depending on the body conditions of the user or the environmental conditions.

### Technical Solution

According to an aspect of the present disclosure, provided is a wrist protecting band attached to a wrist to protect the wrist. The wrist protecting band may include: a first compression member having a first compression pad provided on an inner surface thereof to be able to compress one of an ulna area and a radius area of the wrist toward a central portion of the wrist; a second compression member having a second compression pad provided on an inner surface thereof, in a location opposite to the first compression pad, to be able to compress the other of the ulna area and the radius area of the wrist toward the central portion of the wrist; a connecting member having both ends pivotably coupled to one end portion of the first compression member and to one end portion of the second compression member to connect the first compression member and the second compression member; and a coupling body, wherein the other end portion of the first compression member is pivotably coupled to one end portion of the coupling body, and the other end portion of the second compression member is coupled to or decoupled from the other end portion of the coupling body in response to a user's manipulation. The wrist other than the ulna area and the radius area may not be compressed, and a coupling state of the second compression member may be adjusted by the user's manipulation so that compression load of the first and second compression members on the ulna area and the radius area is adjusted.

The other end portion of the coupling body may have an insertion guide hole, such that the other end portion of the second compression member is able to be inserted into and coupled to the insertion guide hole, and a manipulator operable by a user is disposed in the central portion of the coupling body, the manipulator being configured to be able to adjust a depth to which the second compression member is inserted into the insertion guide hole.

In addition, a separate connecting wire may be coupled to, while penetrating, the other end portion of the second compression member so as to be connected to the manipulator of the coupling body, and the connecting wire may be drawn by a manipulation of the manipulator, so that the second compression member is inserted into and coupled to the insertion guide hole.

In addition, a wire guide may be provided in the insertion guide hole of the coupling body to guide a connection path along which the connecting wire of the second compression member is connected to the manipulator.

In addition, the manipulator may include: a fixed body fixedly coupled to the coupling body, and having a ratchet provided on inner circumferential portions of a top end portion thereof; a rotary body coupled to the fixed body so as to be movable in a top-bottom direction, with a stopper protrusion being provided on one portion of the rotary body to engage with the ratchet to restrain unidirectional rotation during downward movement, wherein the stopper protrusion and the ratchet are disengaged from each other during upward movement; and a dial knob coupled to the rotary body to move in a top-bottom direction and rotate together with the rotary body, wherein the dial knob is manipulated by the user. The connecting wire coupled to, while penetrating, the second compression member may be wound on the rotary body in a single direction.

In addition, the fixed body may be provided with wire inlets to guide the connecting wire along penetration paths, so that the connecting wire is wound on the rotary body by passing through the wire inlets.

In addition, the first compression pad and the second compression pad may be disposed on the first compression member and the second compression member to be movable by predetermined distances in longitudinal directions.

In addition, an insertion protrusion may be provided on each of contact surfaces of the first compression pad and the second compression pad, the contact surfaces being in contact with the first compression member and the second compression member. Each of the first compression member and the second compression member may have a pad receptacle, into which the insertion protrusion is movably inserted, and a movement guide is provided in an internal space of the pad receptacle, such that the insertion protrusion is movable by external force while being engaged with the movement guide.

In addition, auxiliary compression pads may be detachably coupled to contact surfaces of the first compression pad and the second compression pad, the contact surfaces being supposed to be in contact with the wrist.

In addition, each of the contact surfaces of the first compression pad and the second compression pad, to be in contact with the wrist, may have a plurality of fitting holes spaced apart from each other in a longitudinal direction. A fitting protrusion may be provided on each of the first compression pad and the second compression pad, the fitting protrusion being able to be selectively fitted into one of the plurality of fitting holes.

### Advantageous Effects

According to the present disclosure, the wrist protecting band may be used to compress only an ulna area and a radius area of the wrist toward the center of the wrist using compression pads in order to prevent side effects, such as hand tingling or hand swelling. The use of the compression pads can also supplement fixing force of the wrist in order to reliably perform a wrist protecting function of, e.g. preventing the wrist from being excessively bent and reducing pain in the wrist.

In addition, the compression members may be configured to be pivotably coupled to each other and the coupling force between the coupling members is adjusted by a user's manipulation, so that a compression load applied to the ulna area and radius area of the wrist can be adjusted. Accordingly, the wrist protecting band can be more conveniently and optimally used according to user requirements.

In addition, the overall diameter of the wrist protecting band may be increased or reduced using the compression members coupled to each other to be pivotable and a connecting member, so that the process of attaching or detaching the wrist protecting band to or from the wrist can be performed more conveniently. In addition, it is possible to adjust the size of the internal diameter by changing the size of the connecting member without having to change the compression members, thereby facilitating the management of components and the fabrication of the wrist protecting band.

In addition, it is possible to compress or decompress the wrist by pivoting the compression members in a simple manner using the dial-type manipulator while adjusting the strength of compression in a simple manner using the manipulator.

Furthermore, it is possible to adjust the positions and thicknesses of the compression pads compressing the ulna area and the radius area of the wrist according to user requirements, so that the user can more conveniently use the wrist protecting band by modifying the wrist protecting band in an optimal state depending on the body conditions of the user or the environmental conditions.

### DESCRIPTION OF DRAWINGS

FIG. 1 is a perspective view schematically illustrating the shape of a wrist protecting band according to embodiments of the present disclosure;
FIG. 2 is a perspective view schematically illustrating the detached state of the wrist protecting band according to embodiments of the present disclosure;
FIG. 3 schematically illustrates compression load adjusting states of the wrist protecting band according to embodiments of the present disclosure;
FIG. 4 is a cross-sectional view schematically illustrating an internal structure of a manipulating portion of the wrist protecting band according to embodiments of the present disclosure;
FIG. 5 schematically illustrates various shapes of the compression pads of the wrist protecting band according to embodiments of the present disclosure;
FIGS. 6 and 7 schematically illustrate a compression pad moving structure of the wrist protecting band according to embodiments of the present disclosure; and
FIG. 8 schematically illustrates a coupling structure for auxiliary compression pads of the wrist protecting band according to embodiments of the present disclosure.

### MODE FOR INVENTION

Hereinafter, embodiments of the present disclosure will be described in detail with reference to the accompanying drawings. Throughout this document, reference should be made to the drawings, in which the same reference numerals and symbols will be used to designate the same or like components. In the following description of the present disclosure, detailed descriptions of known functions and components incorporated herein will be omitted in the case that the subject matter of the present disclosure may be rendered unclear thereby.

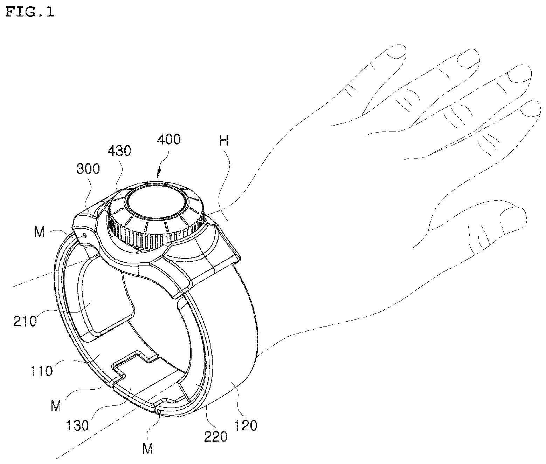

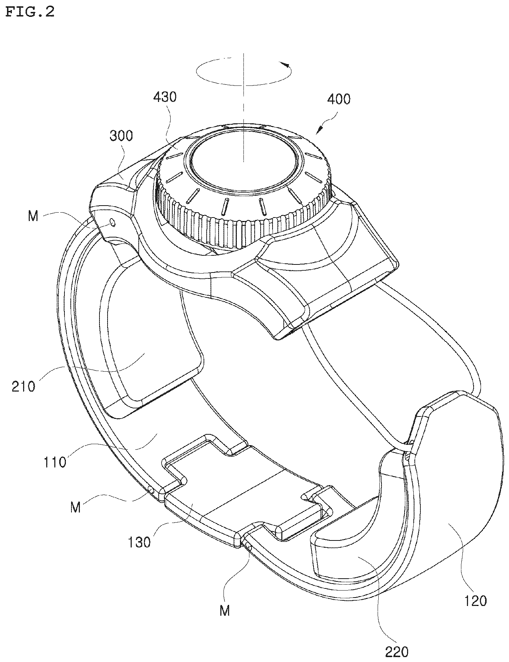

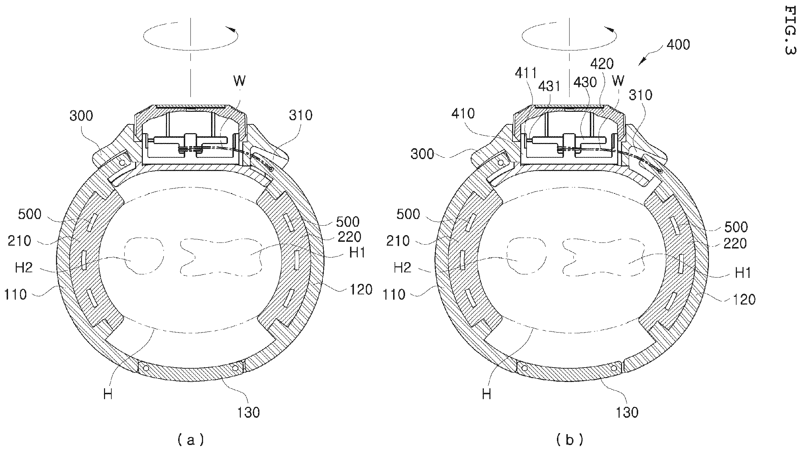

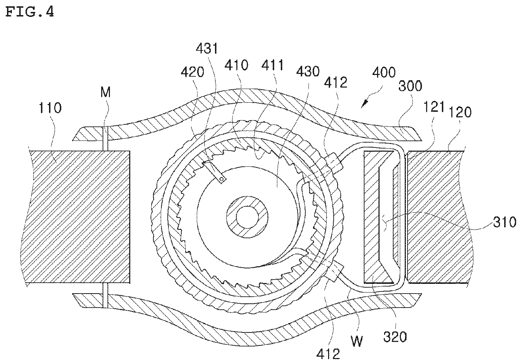

FIG. 1 is a perspective view schematically illustrating the shape of a wrist protecting band according to embodiments of the present disclosure, FIG. 2 is a perspective view schematically illustrating the detached state of the wrist protecting band according to embodiments of the present disclosure, FIG. 3 schematically illustrates compression load adjusting states of the wrist protecting band according to embodiments of the present disclosure, and FIG. 4 is a cross-sectional view schematically illustrating an internal structure of a manipulating portion of the wrist protecting band according to embodiments of the present disclosure.

The wrist protecting band according to embodiments of the present disclosure is configured to be attached to a wrist H of a user to reliably protect a wrist without side effects, such as hand tingling, and includes a first compression member 110, a second compression member 120, a connecting member 130, and a coupling body 300.

The first compression member 110 and the second compression member 120 are configured such that the inner surfaces thereof are concavely curved to surround the wrist H. A first compression pad 210 and a second compression pad 220 are disposed on inner surface portions such that the first compression pad 210 and the second compression pad 220 can compress the area of the ulna H1 and the area of the radius H2 of the wrist toward the center of the wrist. Both end portions of the connecting member 130 are pivotably coupled to one end portion of the first compression member 110 and to one end portion of the second compression member 120 to connect the first compression member 110 and the second compression member 120.

For example, the first compression member 110 may be disposed such that the first compression pad 210 compresses the area of the radius H2 of the wrist toward the center of the wrist while surrounding the area of the radius H2, and the second compression member 120 may be disposed such that the second compression pad 220 compresses the area of the ulna H1 of the wrist H toward the center of the wrist while surrounding the area of the ulna H1. The connecting member 130 is pivotably coupled to one end portion of each of the first compression member 110 and the second compression member 120 to connect the first compression member 110 and the second compression member 120.

The coupling body 300 is configured such that the other end portion of the first compression member 110 is pivotably coupled to one end portion of the coupling body 300 and the other end portion of the second compression member 120 is coupled to or decoupled from the other end portion of the coupling body 300 in response to a user's manipulation.

In the wrist protecting band configured as above, the area of the ulna H1 and the area of the radius H2 of the wrist H are compressed by the first compression pad 210 and the second compression pad 220 of the first compression member 110 and the second compression member 120. Here, the wrist protecting band is configured such that the wrist H, other than the area of the ulna H1 and the area of the radius H2, is not compressed by any portion thereof. That is, the wrist protecting band may be configured such that the portions of the wrist protecting band, other than the area of the ulna H1 and the area of the radius H2, are in simple contact with the wrist H, instead of being spaced apart from the wrist H or compressing the wrist H.

Accordingly, since the area of the ulna H1 and the area of the radius H2 of the wrist H are compressed by the first compression pad 210 and the second compression pad 220, the wrist protecting band according to embodiments of the present disclosure can supplement the fixing force of the wrist H to reliably perform a wrist protecting function of, e.g. preventing the wrist from being excessively bent and reducing pain in the wrist. Here, no portions of the wrist H, other than the area of the ulna H1 and the area of the radius H2, are compressed by the first compression pad 210 and the second compression pad 220 are compressed to obstruct the circulation of blood. Accordingly, no side effect, such as hand tingling or hand swelling, may occur.

In addition, health supplement members 500 formed from zirconium (Zr), germanium (Ge), or the like may be inserted into the first compression pad 210 and the second compression pad 220 in order to apply a variety of health supplement functions to the wrist.

In addition, the first compression member 110 is pivotably coupled to one end portion of the coupling body 300, and the second compression member 120 is disposed to be coupled to or decoupled from the other end portion of the coupling body 300. Here, the second compression member 120 is configured such that the coupling state thereof can be adjusted by the user's manipulation, so that compression load of the first compression member 110 and the second compression member 120 on the area of the ulna H1 and the area of the radius H2 is adjusted.

More specifically, the other end portion of the coupling body 300 has an insertion guide hole 310, such that the other end portion of the second compression member 120 may be inserted into and coupled to the insertion guide hole 310. A manipulator 400 operable by the user is disposed in the central portion of the coupling body 300. The manipulator 400 is configured such that the manipulator 400 may adjust a depth to which the second compression member 120 is inserted into and coupled to the insertion guide hole 310.

Here, a separate connecting wire W may be coupled to, while penetrating, the other end portion of the second compression member 120 so as to be connected to the manipulator 400 of the coupling body 300. The connecting wire W may be drawn by the manipulation of the manipulator 400, so that the second compression member 120 is inserted into the insertion guide hole 310.

According to this configuration, the manipulator 400 of the coupling body 300 may be manipulated to release the connecting wire W from the drawn state, so that the second compression member 120 is decoupled from the coupling body 300 as illustrated in FIG. 1. In contrast, the manipulator 400 of the coupling body 300 may be manipulated to draw the connecting wire W, so that the second compression member 120 may be coupled to the coupling body 300 as illustrated in FIG. 1.

The wrist protecting band as described above constantly maintains the overall shape of a ring, since the second compression member 120 is connected to the coupling body 300 via the connecting wire W even in a case in which the second compression member 120 is decoupled from the coupling body 300. In an open state in which the second compression member 120 is decoupled from the coupling body 300 as illustrated in FIG. 2, the wrist is located in the direction of penetration of the internal space of the wrist protecting band. Afterwards, as illustrated in FIG. 1, it is possible to compress the wrist H by drawing the drawing wire W by manipulating the manipulator 400.

Here, the members pivot about three rotary shafts M, including the rotary shaft M of the coupling body 300 of the first compression member 110, the rotary shaft M of the first compression member 110 of the connecting member 130, and the rotary shaft M of the connecting member 130 of the second compression member 120, the wrist protecting band may expand or contract the entire internal space. Thus, when the wrist H is inserted into or removed from the internal space of the wrist protecting band, the wrist H may be more conveniently moved.

In addition, it may be necessary to use different types of the wrist protecting band depending on the thickness of the wrist H. In this case, it is possible to adjust the internal space of the wrist protecting band to have different diameters by fabricating the first compression member 110 and the second compression member 120 to have the same sizes, fabricating the connecting member 130 may to have different lengths, and coupling the connecting member 130 to the first compression member 110 and the second compression member 120.

In addition, the compression load on the area of the ulna H1 and the area of the radius H2 of the first compression member 110 and the second compression member 120 is adjusted by adjusting the depth to which the second compression member 120 is inserted into the insertion guide hole 310 by the manipulation of the manipulator 400. For example, as illustrated in (a) in FIG. 3, when the manipulator 400 is manipulated so that the second compression member 120 is inserted relatively deeply into the insertion guide hole 310, the compression load of the first compression member 110 and the second compression member 120 on the area of the ulna H1 and the area of the radius H2 relatively increases. As illustrated in (b) in FIG. 3, when the manipulator 400 is manipulated so that the second compression member 120 is inserted relatively shallowly into the insertion guide hole 310, the compression load of the first compression member 110 and the second compression member 120 on the area of the ulna H1 and the area of the radius H2 relatively decreases.

In addition, the manipulator 400 includes a fixed body 410, a rotary body 430, and a dial knob 420. The fixed body 410 is fixedly coupled to the coupling body 300, and has a ratchet 411 provided on the inner circumference of the top end portion of the fixed body 410. The rotary body 430 is coupled to the fixed body 410 so as to be movable in a top-bottom direction. A stopper protrusion 431 provided on one portion of the rotary body 430 engages with the ratchet 411 to restrain unidirectional rotation during downward movement. The engagement of the stopper protrusion 431 and the ratchet 411 is released during upward movement. The dial knob 420 is coupled to the rotary body 430 to move in a top-bottom direction and rotate together with the rotary body 430, and is configured to be manipulated by the user. The connecting wire W coupled to, while penetrating, the second compression member 120 is configured to be wound on the rotary body 430 in a single direction.

Accordingly, in a case in which the dial knob 420 is moved downwardly by pressure, when the dial knob 420 is rotated, for example, in the clockwise direction, the rotary body 430 rotates together with the dial knob 420, and the connecting wire W is wound on the rotary body 430. Accordingly, the connecting wire W is drawn toward the coupling body 300, so that the second compression member 120 is inserted into and coupled to the insertion guide hole 310. In this state, the stopper protrusion 431 is engaged with the ratchet 411 of the fixed body 410, and thus, the rotation of the rotary body 430 in the opposite direction is restrained. Accordingly, in response to the dial knob 420 being manipulated to rotate, the second compression member 120 is continuously and more deeply inserted into the insertion guide hole 310, so that the compression load on the area of the ulna H1 and the area of the radius H2 is increased, and this state is maintained.

In contrast, to reduce the compression load on the area of the ulna H1 and the area of the radius H2 or to decouple the second compression member 120 from the coupling body 300, the dial knob 420 may be drawn upwardly to decouple the stopper protrusion 431 of the rotary body 430 from the ratchet 411 of the fixed body 410. In this state, the second compression member 120 may be rotated and moved in a direction in which the second compression member 120 is decoupled from the coupling body 300, or the dial knob 420 may be rotated in the counterclockwise direction.

In addition, the insertion guide hole 310 of the coupling body 300 may be provided with a wire guide 320 to guide a connection path of the connecting wire W so that the connecting wire W of the second compression member 120 is connected to the manipulator 400 while being wound on the rotary body 430. The wire guide 320 may be provided to guide the connecting wire W, penetrating the second compression member 120, to both side peripheral portions of the insertion guide hole 310.

In addition, the fixed body 410 may be provided with wire inlets 412 to guide the connecting wire W along penetration paths, so that the connecting wire W extending from the second compression member 120 is wound on the rotary body 430 by passing through the wire inlets 412. The wire inlets 412 may be provided on locations adjacent to the both side peripheral portions of the insertion guide hole 310 while having a shape corresponding to the wire guide 320.

Since the arrangement of the connecting wire W is maintained along a predetermined path by the wire guide 320 and the wire inlets 412, the connecting wire W may be reliably maintained in position, without problems, such as twisting or entangling, even in a case in which the second compression member 120 is coupled to or decoupled from the coupling body 300.

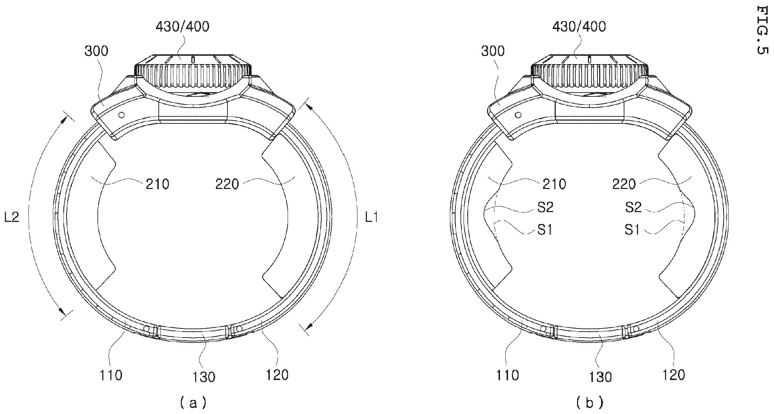

FIG. 5 schematically illustrates various shapes of the compression pads of the wrist protecting band according to embodiments of the present disclosure.

As illustrated in FIG. 5, the first compression pad 210 and the second compression pad 220 according to embodiments of the present disclosure may be provided with a variety of shapes.

For example, as illustrated in (a) in FIG. 5, the length L1 of the second compression pad 220 that compresses the area of the ulna H1 may be greater than the length L2 of the first compression pad 210 that compresses the area of the radius H2. The bones constituting an arm include the ulna H1 and the radius H2. In the wrist H, the ulna H1 and the radius H2 protrude outwardly. The ulna H1 is thicker than the radius H2, and has a wider protruding area in the wrist H than the radius H2. Since the area of the ulna H1 is relatively wider, it may be more appropriate to compress the area of the ulna H1 and the area of the radius H2 if the length of the second compression pad 220 compressing the area of the ulna H1 is set to be greater.

In addition, as illustrated in (b) in FIG. 5, the first compression pad 210 and the second compression pad 220 may be configured such that surfaces of longitudinally intermediate portions thereof, to be in contact with the wrist H, may be concavely curved. That is, in a case in which the first compression pad 210 and the second compression pad 220 have a uniform thickness, the surfaces to be in contact with the wrist H may be formed along lines S1 to be parallel to the curves of the inner surfaces of the first compression member 110 and the second compression member 120. In contrast, according to embodiments of the present disclosure, the surfaces to be in contact with the wrist H may be formed along lines S2 having a concavely curved shape. Since the surfaces to be in contact with the wrist H are formed to be concavely curved, the first compression pad 210 and the second compression pad 220 may be more closely in contact with the wrist H, thereby more reliably compressing the ulna area and the radius area of the wrist H.

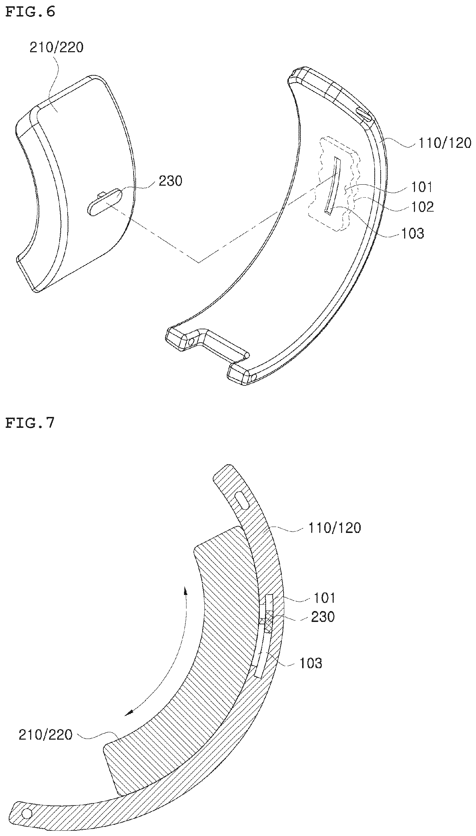

FIGS. 6 and 7 schematically illustrate a compression pad moving structure of the wrist protecting band according to embodiments of the present disclosure.

As illustrated in FIGS. 6 and 7, the first compression pad 210 and the second compression pad 220 according to embodiments of the present disclosure may be disposed to be movable by predetermined distances in the longitudinal directions of the first compression member 110 and the second compression member 120. Accordingly, the positions of the first compression pad 210 and the second compression pad 220 may be adjusted, so that the areas of the ulna and the radius, which may differ depending on the user, can be accurately compressed.

When the wrist protecting band is attached to the wrist H, the first compression pad 210 and the second compression pad 220 are disposed opposite to each other to compress the area of the ulna H1 and the area of the radius H2 of the wrist H. Here, since the shape of the wrist H, the positions of the ulna H1 and the radius H2, and the like, may differ depending on the user, the first compression pad 210 and the second compression pad 220 may not accurately compress intended points of some users.

Accordingly, the user is allowed to adjust the positions of the first compression pad 210 and the second compression pad 220 to be suitable to him or her by moving the first compression pad 210 and the second compression pad 220. In this manner, the user can locate the first compression pad 210 and the second compression pad 220 at correct positions, thereby correctly compress the area of the ulna H1 and the area of the radius H2.

To move the positions of the first compression pad 210 and the second compression pad 220, each of the surfaces of the first compression pad 210 and the second compression pad 220, in contact with the first compression member 110 and the second compression member 120, may be provided with an insertion protrusion 230. The insertion protrusions 230 may be inserted into the internal space of each of the first compression member 110 and the second compression member 120. In addition, each of the first compression member 110 and the second compression member 120 is provided with a pad receptacle 101, into which the insertion protrusion 230 is movably inserted. A movement guide 102 is provided in the internal space of the pad receptacle 101, such that the insertion protrusion 230 may be moved by external force while being engaged with movement guide 102. Here, an open portion 103 may be provided in the inner side of each of the first compression member 110 and the second compression member 120 to extend in the longitudinal direction, such that the insertion protrusion 230 of each of the first compression pad 210 and the second compression pad 220 is inserted through the open portion 103. The insertion protrusion 230 may be inserted into the pad receptacle 101 through the open portion 103 to be accommodated in the pad receptacle 101, and may move in the longitudinal direction along the movement guide 102 disposed within the pad receptacle 101. Concave-convex portions may be provided on peripheral portions of the movement guide 102, and the insertion protrusion 230 may be moved by external force in a stepwise manner while being engaged with the concave-convex portions.

According to the above-described configuration, in a case in which the user has the wrist protecting band attached to the wrist H, the user can adjust the positions of the first compression pad 210 and the second compression pad 220 by pushing or pulling the first compression pad 210 and the second compression pad 220 in the longitudinal direction, thereby accurately compressing the ulna area and the radius area.

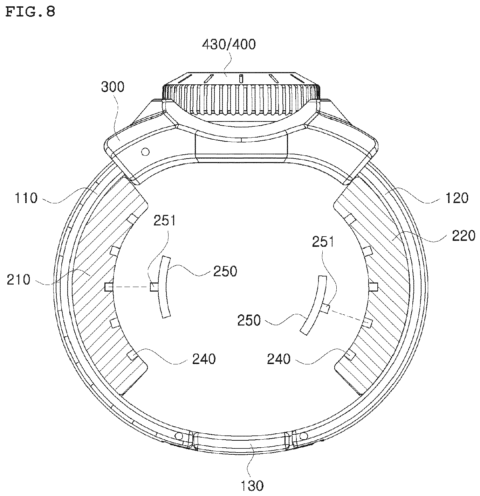

FIG. 8 schematically illustrates a coupling structure for auxiliary compression pads of the wrist protecting band according to embodiments of the present disclosure.

As illustrated in FIG. 8, the first compression pad 210 and the second compression pad 220 according to embodiments of the present disclosure may further include auxiliary compression pads 250.

The auxiliary compression pads 250 may be configured such that the auxiliary compression pads 250 are detachably coupled to the surfaces of the first compression pad 210 and the second compression pad 220, the surfaces being supposed to be in contact with the wrist H. The auxiliary compression pads 250 are configured to be coupled to the surfaces of the first compression pad 210 and the second compression pad 220, the surfaces being supposed to be in contact with the wrist H, such that the auxiliary compression pads 250 substantially compress the area of the ulna H1 and the area of the radius H2 of the wrist H. The auxiliary compression pads 250 may be made of a soft material, such as leather or silicone.

It is possible to adjust the thicknesses of the first compression pad 210 and the second compression pad 220 by coupling or decoupling the auxiliary compression pads 250 to or from the first compression pad 210 and the second compression pad 220. That is, coupling the auxiliary compression pads 250 to the first compression pad 210 and the second compression pad 220 increases the overall thicknesses of the first compression pad 210 and the second compression pad 220, while decoupling the auxiliary compression pads 250 from the first compression pad 210 and the second compression pad 220 reduces the overall thicknesses of the first compression pad 210 and the second compression pad 220.

As described above, it is possible to adjust the overall thicknesses of the first compression pad 210 and the second compression pad 220 using the auxiliary compression pads 250. Accordingly, the user can adjust the thicknesses of the first compression pad 210 and the second compression pad 220 depending on the body conditions of the user or the environmental conditions.

Here, the surfaces of the first compression pad 210 and the second compression pad 220, to be in contact with the wrist H, may have fitting holes 240. A fitting protrusion 251 may be provided on one surface of each of the auxiliary compression pads 250 so as to be fitted into the fitting holes 240. In response to the coupling of the fitting protrusions 251 and the fitting holes 240, the auxiliary compression pads 250 may be detachably coupled to the first compression pad 210 and the second compression pad 220.

In addition, it is possible to adjust the positions of the portions substantially compressing the ulna area and the radius area by adjusting the locations to which the auxiliary compression pads 250 are coupled. In this regard, the plurality of coupling holes 240 may be provided in each of the first compression pad 210 and the second compression pad 220 in the longitudinal direction.

That is, it is possible to adjust the coupling positions of the auxiliary compression pads 250 by may selectively coupling the fitting protrusion 251 of each of the auxiliary compression pads 250 to one of the plurality of coupling holes 240 in each of the first compression pad 210 and the second compression pad 220, as illustrated in FIG. 8. This structure makes it possible to conveniently adjust the locations of the wrist H to be compressed by the auxiliary compression pads 250.

The foregoing descriptions have been presented in order to explain certain principles of the present disclosure by way of example. A person having ordinary knowledge in the art to which the present disclosure relates could make various modifications and variations without departing from the essential features of the present disclosure. The foregoing embodiments disclosed herein shall be interpreted as being illustrative, while not being limitative, of the principle and scope of the present disclosure. It should be understood that the scope of the invention is defined by the appended Claims

## Claims

1. A wrist protecting band configured to be attached to a wrist (H), the wrist protecting band comprising:
a first compression member (110) having a first compression pad (210) provided on an inner surface thereof to be able to compress one of an ulna area (H1) and a radius area (H2) of the wrist (H) toward a central portion of the wrist (H);
a second compression member (120) having a second compression pad (220) provided on an inner surface thereof, in a location opposite to the first compression pad (210), to be able to compress the other of the ulna area and the radius area of the wrist (H) toward the central portion of the wrist (H); **characterised in that** it further comprises
a connecting member (130) having both ends pivotably coupled to one end portion of the first compression member (110) and to one end portion of the second compression member (120) to connect the first compression member (110) and the second compression member (120); and
a coupling body (300), wherein the other end portion of the first compression member (110) is pivotably coupled to one end portion of the coupling body (300), and the other end portion of the second compression member (120) is coupled to or decoupled from the other end portion of the coupling body (300) in response to a user's manipulation,
wherein the wrist (H) other than the ulna area and the radius area is not compressed, and a coupling state of the second compression member (120) is adjustable by the user's manipulation so that compression load of the first and second compression members (110, 120) on the ulna area and the radius area is adjusted.

2. The wrist protecting band according to claim 1, wherein the other end portion of the coupling body (300) has an insertion guide hole (310), such that the other end portion of the second compression member (120) is able to be inserted into and coupled to the insertion guide hole (310), and
a manipulator (400) operable by a user is disposed in the central portion of the coupling body (300), the manipulator (400) being configured to be able to adjust a depth to which the second compression member (120) is inserted into the insertion guide hole (310).

3. The wrist protecting band according to claim 2, wherein a separate connecting wire (W) is coupled to, while penetrating, the other end portion of the second compression member (120) so as to be connected to the manipulator (400) of the coupling body (300), and
the connecting wire (W) is drawn by a manipulation of the manipulator (400), so that the second compression member (120) is inserted into and coupled to the insertion guide hole (310).

4. The wrist protecting band according to claim 3, wherein a wire guide (320) is provided in the insertion guide hole (310) of the coupling body (300) to guide a connection path along which the connecting wire (W) of the second compression member (120) is connected to the manipulator (400).

5. The wrist protecting band according to claim 4, wherein the manipulator (400) includes:
a fixed body (410) fixedly coupled to the coupling body (300), and having a ratchet (411) provided on inner circumferential portions of a top end portion thereof;
a rotary body (430) coupled to the fixed body (410) so as to be movable in a top-bottom direction, with a stopper protrusion (431) being provided on one portion of the rotary body (430) to engage with the ratchet (411) to restrain unidirectional rotation during downward movement,
wherein the stopper protrusion (431) and the ratchet (411) are disengaged from each other during upward movement; and
a dial knob (420) coupled to the rotary body (430) to move in a top-bottom direction and rotate together with the rotary body (430), wherein the dial knob (420) is manipulated by the user,
wherein the connecting wire (W) coupled to, while penetrating, the second compression member (120) is wound on the rotary body (430) in a single direction.

6. The wrist protecting band according to claim 5, wherein the fixed body (410) is provided with wire inlets (412) to guide the connecting wire (W) along penetration paths, so that the connecting wire (W) is wound on the rotary body (430) by passing through the wire inlets (412).

7. The wrist protecting band according to any one of claims 1 to 6, wherein the first compression pad (210) and the second compression pad (220) are disposed on the first compression member (110) and the second compression member (120) to be movable by predetermined distances in longitudinal directions.

8. The wrist protecting band according to claim 7, wherein an insertion protrusion (230) is provided on each of contact surfaces of the first compression pad (210) and the second compression pad (220), the contact surfaces being in contact with the first compression member (210) and the second compression member (220), and
each of the first compression member (110) and the second compression member (120) has a pad receptacle (101), into which the insertion protrusion (230) is movably inserted, and a movement guide (102) is provided in an internal space of the pad receptacle (101), such that the insertion protrusion (230) is movable by external force while being engaged with the movement guide(102).

9. The wrist protecting band according to any one of claims 1 to 6, wherein auxiliary compression pads (250) are detachably coupled to contact surfaces of the first compression pad (210) and the second compression pad (220), the contact surfaces being supposed to be in contact with the wrist (H).

10. The wrist protecting band according to claim 9, wherein each of the contact surfaces of the first compression pad (210) and the second compression pad (220), to be in contact with the wrist (H), has a plurality of fitting holes (240) spaced apart from each other in a longitudinal direction, and
a fitting protrusion (251) is provided on each of the first compression pad (210) and the second compression pad (220) , the fitting protrusion (251) being able to be selectively fitted into one of the plurality of fitting holes (240).

## Patentansprüche

1. Handgelenkschutzband, das konfiguriert ist, an einem Handgelenk (H) befestigt zu werden, wobei das Handgelenkschutzband Folgendes umfasst:
ein erstes Kompressionselement (110), das ein erstes Kompressionspolster (210) aufweist, das auf einer inneren Oberfläche davon vorgesehen ist, um entweder einen Ellenbereich (H1) und einen Speichenbereich (H2) des Handgelenks (H) in Richtung eines zentralen Abschnitts des Handgelenks (H) komprimieren zu können,
ein zweites Kompressionselement (120), das ein zweites Kompressionspolster (220) aufweist, das auf einer inneren Oberfläche davon vorgesehen ist, an einem Ort, der dem ersten Kompressionspolster (210) gegenüberliegt, um den anderen des Ellenbereichs und des Speichenbereichs des Handgelenks (H) in Richtung des zentralen Abschnitts des Handgelenks (H) komprimieren zu können, **dadurch gekennzeichnet, dass** es ferner Folgendes umfasst:
ein Verbindungselement (130), dessen beide Enden an einem Endabschnitt des ersten Kompressionselements (110) und an einem Endabschnitt des zweiten Kompressionselements (120) schwenkbar gekoppelt sind, um das erste Kompressionselement (110) und das zweite Kompressionselement (120) zu verbinden; und
einen Kopplungskörper (300), wobei der andere Endabschnitt des ersten Kompressionselements (110) an einem Endabschnitt des Kopplungskörpers (300) schwenkbar gekoppelt ist und der andere Endabschnitt des zweiten Kompressionselements (120) als Reaktion auf eine Handhabung des Benutzers an den anderen Endabschnitt des Kopplungskörpers (300) gekoppelt oder davon abgekoppelt wird,
wobei das Handgelenk (H) mit Ausnahme des Ellenbereichs und des Speichenbereichs nicht komprimiert wird und ein Kopplungszustand des zweiten Kompressionselements (120) durch die Handhabung des Benutzers derart eingestellt werden kann, dass die Kompressionslast des ersten und des zweiten Kompressionselements (110, 120) auf den Ellenbereich und den Speichenbereich eingestellt wird.

2. Handgelenkschutzband nach Anspruch 1, wobei der andere Endabschnitt des Kopplungskörpers (300) ein Einführführungsloch (310) aufweist, derart, dass der andere Endabschnitt des zweiten Kompressionselements (120) in das Einführführungsloch (310) eingeführt und daran gekoppelt werden kann, und
eine Handhabungsvorrichtung (400), die durch einen Benutzer bedient werden kann, in dem zentralen Abschnitt des Kopplungskörpers (300) angeordnet ist, wobei die Handhabungsvorrichtung (400) konfiguriert ist, eine Tiefe einstellen zu können, bis zu der das zweite Kompressionselement (120) in das Einführführungsloch (310) eingeführt wird.

3. Handgelenkschutzband nach Anspruch 2, wobei ein separater Verbindungsdraht (W) während des Eindringens an den anderen Endabschnitt des zweiten Kompressionselements (120) gekoppelt wird, um mit der Handhabungsvorrichtung (400) des Kopplungskörpers (300) verbunden zu werden, und
der Verbindungsdraht (W) durch eine Handhabung der Handhabungsvorrichtung (400) derart gezogen wird, dass das zweite Kompressionselement (120) in das Einführführungsloch (310) eingeführt und daran gekoppelt wird.

4. Handgelenkschutzband nach Anspruch 3, wobei eine Drahtführung (320) in dem Einführführungsloch (310) des Kopplungskörpers (300) vorgesehen ist, um einen Verbindungspfad, entlang dessen der Verbindungsdraht (W) des zweiten Kompressionselements (120) mit der Handhabungsvorrichtung (400) verbunden wird, zu führen.

5. Handgelenkschutzband nach Anspruch 4, wobei die Handhabungsvorrichtung (400) Folgendes umfasst:
einen festen Körper (410), der mit dem Kopplungskörper (300) fest gekoppelt ist, und eine Sperrvorrichtung (411) aufweist, die an Innenumfangsabschnitten eines inneren Endabschnitts davon vorgesehen ist,
einen Drehkörper (430), der an den festen Körper (410) gekoppelt ist, um in einer Oben-Unten-Richtung bewegt werden zu können, mit einem Anschlagvorsprung (431), der auf einem Abschnitt des Drehkörpers (430) vorgesehen ist, um mit der Sperrvorrichtung (411) in Eingriff zu gelangen, um während der Abwärtsbewegung eine unidirektionale Drehung zu verhindern,
wobei der Anschlagvorsprung (431) und die Sperrvorrichtung (411) während der Aufwärtsbewegung voneinander gelöst werden, und
einen Drehknopf (420), der an den Drehkörper (430) gekoppelt ist, um sich in einer Oben-Unten-Richtung zu bewegen, und sich zusammen mit dem Drehkörper (430) zu drehen, wobei der Drehknopf (420) durch den Benutzer gehandhabt wird,
wobei der Verbindungsdraht (W), der, während des Eindringens, an das zweite Kompressionselement (120) gekoppelt ist, in einer einzigen Richtung um den Drehkörper (430) gewickelt wird.

6. Handgelenkschutzband nach Anspruch 5, wobei der feste Körper (410) mit Drahteinlässen (412) versehen ist, um den Verbindungsdraht (W) entlang Eindringpfaden derart zu führen, dass der Verbindungsdraht (W) auf den Drehkörper (430) gewickelt wird, indem er durch die Drahteinlässe (412) hindurch läuft.

7. Handgelenkschutzband nach einem der Ansprüche 1 bis 6, wobei das erste Kompressionspolster (210) und das zweite Kompressionspolster (220) auf dem ersten Kompressionselement (110) und dem zweiten Kompressionselement (120) angeordnet sind, um durch vorgegebene Entfernungen in Längsrichtungen bewegt werden zu können.

8. Handgelenkschutzband nach Anspruch 7, wobei ein Einführvorsprung (230) auf jeder der Kontaktflächen des ersten Kompressionspolsters (210) und des zweiten Kompressionspolsters (220) vorgesehen ist, wobei sich die Kontaktflächen in Kontakt mit dem ersten Kompressionselement (110) und dem zweiten Kompressionselement (120) befinden, und
jedes des ersten Kompressionselements (110) und des zweiten Kompressionselements (120) eine Polsteraufnahme (101) aufweist, in die der Einführvorsprung (230) beweglich eingeführt wird, und eine Bewegungsführung (102) in einem Innenraum der Polsteraufnahme (101) derart vorgesehen ist, dass der Einführvorsprung (230) durch äußere Kraft bewegt werden kann, während er sich in Eingriff mit der Bewegungsführung (102) befindet.

9. Handgelenkschutzband nach einem der Ansprüche 1 bis 6, wobei Hilfs-Kompressionspolster (250) an den Kontaktflächen des ersten Kompressionspolsters (210) und des zweiten Kompressionspolsters (220) lösbar gekoppelt sind, wobei die Kontaktflächen mit dem Handgelenk (H) in Kontakt sein sollen.

10. Handgelenkschutzband nach Anspruch 9, wobei jede der Kontaktflächen des ersten Kompressionspolsters (210) und des zweiten Kompressionspolsters (220), die in Kontakt mit dem Handgelenk (H) sein sollen, mehrere Passlöcher (240), die voneinander in einer Längsrichtung beabstandet sind, aufweist, und
ein Passungsvorsprung (251) auf jedem des ersten Kompressionspolsters (210) und des zweiten Kompressionspolsters (220) vorgesehen ist, wobei der Passungsvorsprung (251) wahlweise in eines der mehreren Passlöcher (240) eingepasst werden kann.

## Revendications

1. Bande de protection de poignet configurée pour être attachée à un poignet (H), la bande de protection de poignet comportant :
un premier élément de compression (110) ayant un premier tampon de compression (210) agencé sur une surface intérieure de celui-ci pour pouvoir comprimer une zone parmi une zone d'ulna (H1) et une zone de radius (H2) du poignet (H) vers une partie centrale du poignet (H) ;
un second élément de compression (120) ayant un second tampon de compression (220) fourni sur une surface intérieure de celui-ci, à un emplacement opposé au premier tampon de compression (210), pour pouvoir comprimer l'autre zone parmi la zone d'ulna et la zone de radius du poignet (H) vers la partie centrale du poignet (H) ; **caractérisée en ce qu'**elle comporte en outre :
un élément de liaison (130) ayant les deux extrémités couplées à pivotement à une partie d'extrémité du premier élément de compression (110) et à une partie d'extrémité du second élément de compression (120) pour relier le premier élément de compression (110) et le second élément de compression (120) ; et
un corps de couplage (300), dans lequel l'autre partie d'extrémité du premier élément de compression (110) est couplée à pivotement à la partie d'extrémité du corps de couplage (300), et l'autre partie d'extrémité du second élément de compression (120) est couplée à l'autre partie d'extrémité du corps de couplage (300) ou est découplée de celle-ci en réponse à une manipulation de l'utilisateur,
dans laquelle le poignet (H) autre que la zone d'ulna et la zone de radius n'est pas comprimé, et un état de couplage du second élément de compression (120) peut être réglé par la manipulation de l'utilisateur de sorte qu'une charge de compression des premier et second éléments de compression (110, 120) sur la zone d'ulna et la zone de radius est réglée.

2. Bande de protection de poignet selon la revendication 1, dans laquelle l'autre partie d'extrémité du corps de couplage (300) a un trou de guidage d'insertion (310), de telle sorte que l'autre partie d'extrémité du second élément de compression (120) peut être insérée dans le trou de guidage d'insertion (310) et être couplée à celui-ci, et
un manipulateur (400) pouvant être actionné par un utilisateur est disposé dans la partie centrale du corps de couplage (300), le manipulateur (400) étant configuré pour pouvoir régler une profondeur jusqu'à laquelle le second élément de compression (120) est inséré dans le trou de guidage d'insertion (310).

3. Bande de protection de poignet selon la revendication 2, dans laquelle un fil de liaison séparé (W) est couplé à l'autre partie d'extrémité du second élément de compression (120), tout en pénétrant dans celle-ci, de manière à être relié au manipulateur (400) du corps de couplage (300), et
le fil de liaison (W) est tiré par une manipulation du manipulateur (400), de sorte que le second élément de compression (120) est inséré dans le trou de guidage d'insertion (310) et couplé à celui-ci.

4. Bande de protection de poignet selon la revendication 3, dans laquelle un guide-fil (320) est agencé dans le trou de guidage d'insertion (310) du corps de couplage (300) pour guider un trajet de liaison le long duquel le fil de liaison (W) du second élément de compression (120) est relié au manipulateur (400).

5. Bande de protection de poignet selon la revendication 4, dans laquelle le manipulateur (400) inclut :
un corps fixe (410) fixement couplé au corps de couplage (300), et ayant un encliquetage (411) agencé sur des parties circonférentielles intérieures d'une partie d'extrémité supérieure de celui-ci ;
un corps rotatif (430) couplé au corps fixe (410) de manière à être mobile dans une direction vers le haut-bas, avec une saillie de butée (431) étant agencée sur une partie du corps rotatif (430) pour venir en prise avec l'encliquetage (411) pour empêcher un mouvement unidirectionnel pendant un mouvement vers le bas,
dans lequel la saillie de butée (431) et l'encliquetage (411) sont désengagés l'un de l'autre pendant un mouvement vers le haut ; et
un bouton-cadran (420) couplé au corps rotatif (430) pour se déplacer dans une direction vers le haut-bas et pour tourner en association avec le corps rotatif (430), dans lequel le bouton-cadran (420) est manipulé par l'utilisateur,
dans lequel le fil de liaison (W) couplé au second élément de compression (120), tout en pénétrant dans celui-ci, est enroulé sur le corps rotatif (430) dans un seul sens.

6. Bande de protection de poignet selon la revendication 5, dans laquelle le corps fixe (410) est pourvu d'entrées de fil (412) pour guider le fil de liaison (W) le long de trajets de pénétration, de sorte que le fil de liaison (W) est enroulé sur le corps rotatif (430) en passant à travers les entrées de fil (412).

7. Bande de protection de poignet selon l'une quelconque des revendications 1 à 6, dans laquelle le premier tampon de compression (210) et le second tampon de compression (220) sont disposés sur le premier élément de compression (110) et le second élément de compression (120) pour pouvoir être déplacés sur des distances prédéterminées dans des directions longitudinales.

8. Bande de protection de poignet selon la revendication 7, dans laquelle une saillie d'insertion (230) est agencée sur chacune des surfaces de contact du premier tampon de compression (210) et du second tampon de compression (220), les surfaces de contact étant en contact avec le premier élément de compression (210) et le second élément de compression (220), et
chaque élément parmi le premier élément de compression (110) et le second élément de compression (120) a un logement de tampon (101), dans lequel la saillie d'insertion (230) est insérée de manière mobile, et un guide de mouvement (102) est agencé dans un espace interne du logement de tampon (101), de telle sorte que la saillie d'insertion (230) peut être déplacée par une force externe tout en étant en prise avec le guide de mouvement (102).

9. Bande de protection de poignet selon l'une quelconque des revendications 1 à 6, dans laquelle des tampons de compression auxiliaires (250) sont couplés de manière détachable à des surfaces de contact du premier tampon de compression (210) et du second tampon de compression (220), les surfaces de contact étant supposées être en contact avec le poignet (H).

10. Bande de protection de poignet selon la revendication 9, dans laquelle chacune des surfaces de contact du premier tampon de compression (210) et du second tampon de compression (220), devant être en contact avec le poignet (H), a une pluralité de trous de montage (240) espacés les uns des autres dans une direction longitudinale, et
une saillie de montage (251) est agencée sur chaque tampon parmi le premier tampon de compression (210) et le second tampon de compression (220), les saillies de montage (251) pouvant être sélectivement montées dans un trou de la pluralité de trous de montage (240).
